# EUROPÄISCHE PATENTSCHRIFT

(11) **EP 0 420 800 B1**
(45) Veröffentlichungstag und Bekanntmachung des Hinweises auf die Patenterteilung: **09.03.1994**
(21) Anmeldenummer: 90810698.2
(22) Anmeldetag: 14.09.1990
(51) Int. Cl.: G01N 1/10, A01J 5/04

(54) **Verfahren zum Entnehmen einer Milchprobe**
Method for taking a milk sample
Procédé pour la prise d'un échantillon de lait

(30) Priorität: 25.09.1989 CH 3473/89
(43) Veröffentlichungstag der Anmeldung: 03.04.1991
(73) Patentinhaber: ULTRAKUST electronic GmbH, 94239 Gotteszell (DE)
(72) Erfinder:
(74) Vertreter: Heim, Hans-Karl, Dipl.-Ing.

(56) Entgegenhaltungen:
- DE-A- 3 502 858
- US-A- 3 184 973
- US-A- 4 501 161
- DMZ DEUTSCHE MOLKEREI-ZEITUNG, Nr. 44, Oktober 1984, Seiten 1504-1510; R. LANDRE: "Grenzen der Entwicklung automatischer Probenahmegeräte"

## Beschreibung

Diese Erfindung betrifft ein Verfahren zum Entnehmen einer Milchprobe aus einer Milchförderleitung gemäss dem Patentanspruch 1, eine Vorrichtung an einer Milchförderleitung zum Durchführen des Verfahrens gemäss dem Patentanspruch 5 sowie eine Probeentnahmeeinrichtung, insbesondere an Milchsammelwagen gemäss Patentanspruch 12.

Die Anforderungen an automatische Einrichtungen zur Milchprobeerfassung sind hoch. Es wird eine einwandfreie Funktion vorausgesetzt, so dass Verschleppungsfehler minimal sind und die Proben in mengenmässig richtigem Verhältnis genommen werden. Ein Qualitäts-Bezahlungssystem, wo Milchproben von Hand mit Rührer und Kelle gefasst werden, ist nicht über alle Zweifel erhaben, indem beispielsweise Verwechslungsfehler vorkommen können oder Probefassung und Milchannahme zeitlich auseinanderliegen.

An den benetzten Innenflächen bei einer Einrichtung zur automatischen Probenahme bleibt nach jedem Probenahmevorgang ein gewisser Rest an Milch als Haft- und als Akkumulationsflüssigkeit zurück. Diese Restmilch wird durch den nachfolgenden Probenahmevorgang wieder abgespült, mit der nachfolgenden Milch vermischt und teilweise in der Probeflasche als sogenannte Verschleppung mitgeführt. Durch besondere, konstruktive und verfahrenstechnische Massnahmen an automatisierten Einrichtungen zur Milchprobeerfassung können Verschleppungsfehler minimalisiert werden.

Im weiteren ist wichtig, dass repräsentative Proben entnommen werden, wobei eine repräsentative Probe als Teilmenge in den zu untersuchenden Merkmalen dem Durchschnitt der Gesamtmenge, aus der die Probe entnommen worden ist, entspricht. Ebenfalls durch konstruktive und verfahrenstechnische Merkmale ist es möglich, die Repräsentativität hochzuhalten.

Eine bekannte Ausführungsform einer Vorrichtung zur unmittelbaren Ueberführung von Milchteilmengen aus einer Milchförderleitung in eine Probeflasche ist in der deutschen Offenlegungsschrift DE 36 27 849 offenbart. Diese Vorrichtung funktioniert derart, dass eine Probeflasche über ein elastisches Verschlussteil form- und/oder kraftschlüssig an einer peripheren Oeffnung der Milchförderleitung dichtend befestigt ist. Je eine in der Förderleitung angeordnete Füll- und Entlüftungsnadel sind quer zur Förderleitung bewegbar und zur Probeentnahmestellung in die Probeflasche einführbar. Vor dem Einführen in die Probenahmeflasche befinden sich die Nadeln in der sogenannten Spülstellung im Milchstrom der Förderleitung. Mittels einem, ebenfalls in die Milchförderleitung ragenden und in dieser bewegbar angeordneten Schliessglied kann die Probeentnahme zeitlich gesteuert werden. Die Nachteile dieser bekannten Konstruktion sind einerseits darin zu sehen, dass sich bewegbare, schwer zu reinigende Teile in der Milchförderleitung befinden, was nebst den bereits angesprochen Reinigungsproblemen andererseits Abdichtungsprobleme aufwirft. Bei sogenannten Vakuumschlägen beim Fördern der Milch in der Milchförderleitung kann Milch aus der Probenahmeflasche in die Milchförderleitung zurückfliessen. Dies beeinträchtigt eine repräsentative Probeentnahme.

Eine weitere bekannte Ausführungsform einer Vorrichtung für die Entnahme von Milchproben zeigt die deutsche Offenlegungsschrift DE 35 02 858. Die Vorrichtung umfasst eine in die Milchförderleitung eingeführte Abzweigleitung, durch die mittels einer Schlauchpumpe, die mit Impulsen getaktet angetrieben ist, Milch in die Probeflasche abgezweigt wird. Je nach der zu erwartenden Milchmenge kann zum Erreichen einer repräsentativen Probe die Impulslänge und/oder die Impulsfrequenz verändert werden. Die Verschleppung von Milch aus einem vorhergegangenen Probeentnahmevorgang in die nächste Probe wird dadurch verhindert, dass sobald die Förderleitung mit Milch gefüllt ist, automatisch der Probenabweig für eine kurze Zeit betätigt wird, um den Milchabzweigweg von anhaftenden Milchresten freizuspülen, wobei diese Spülmilch nicht in die Probeflasche sondern abgeleitet wird. Nachteilige, konstruktive Zusatzaufwendungen sind bei dieser Vorrichtung zum Betätigen des Probeabzweiges und zum Fassen der Spülmilch, was mittels einem Trichter geschieht, notwendig.

Es ist die Aufgabe der vorliegenden Erfindung, die obgenannten Nachteile zu beseitigen. Erfingungsgemäss wird nun ein Verfahren zum Entnehmen einer Milchprobe aus einer Milchförderleitung vorgeschlagen, das durch die im Patentanspruch 1 aufgeführten Merkmale gekennzeichnet ist. Eine Vorrichtung an einer Milchförderleitung zum Durchführen des Verfahrens ist durch die Merkmale des Patentanspruches 5 gekennzeichnet. Besondere Merkmale einer Probenahmeeinrichtung, insbesondere an Milchsammelwagen, mit einer erfindungsgemässen Vorrichtung sind im Patentanspruch 12 aufgeführt.

Mit dem vorgeschlagenen Verfahren und der Vorrichtung zum Durchführen des Verfahrens kann auf leicht durchführbare, kostengünstige Art die Vorrichtung von der Restmilch des Vorlieferanten befreit werden. Die Vorrichtung ist im Aufbau einfach, besondere Dichtungs- und Reinigungsprobleme sind nicht vorhanden. Bevorzugte Verfahrensschritte und Ausführungsformen der Vorrichtung sind in den Patentansprüchen 2 bis 4, bzw. 6 bis 11 aufgeführt. Durch die Merkmale der Probenahmeeinrichtung kann das Entnehmen einer nicht repräsentativen Probe praktisch ausgeschlossen werden.

Anhand eines Ausführungsbeispieles soll die Erfindung zusammen mit Figuren näher beschrieben werden. Es zeigen:
- Fig. 1: eine erfindungsgemässe Vorrichtung an einer Milchförderleitung und
- Fig. 2: die wesentlichsten Teile einer Probenahmeeinrichtung, insbesondere an Milchsammelwagen mit einer Vorrichtung gemäss der Fig. 1.

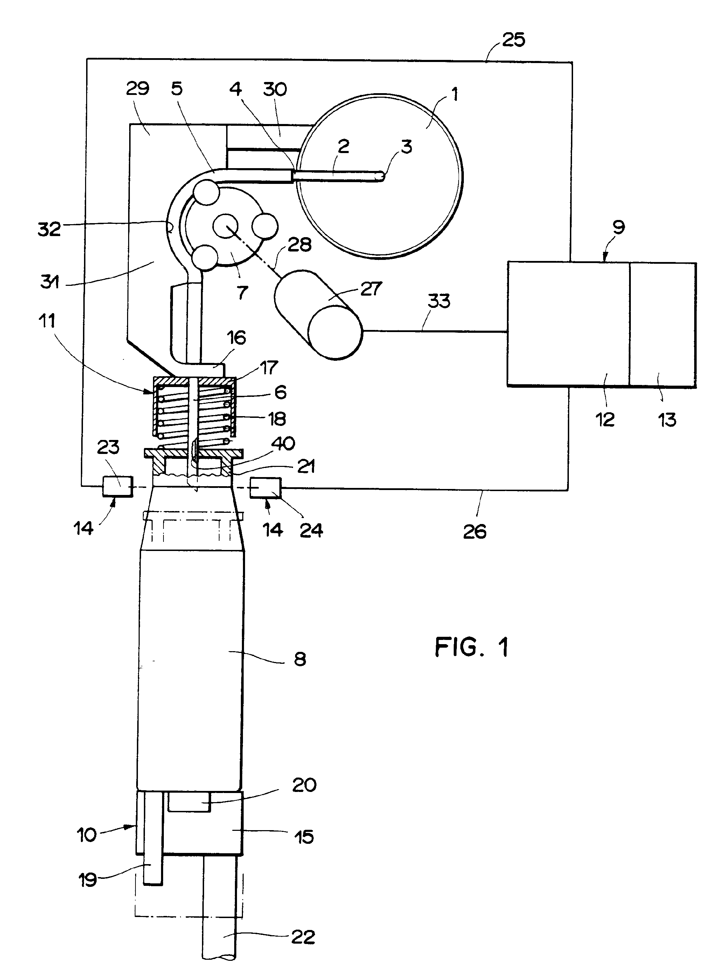

In der Fig. 1 ist mit den Bezugszeichen 1 eine im Schnitt dargestellte Milchförderleitung gekennzeichnet, die zum Verbinden eines nicht gezeigten Milchlagertankes eines Milchlieferanten mit einem Milchsammelwagen bestimmt ist. In die Milchförderleitung 1 ist radial ein Absaugstutzen 2 eingeführt, dessen in die Milchförderleitung 1 ragendes Ende 3 gegen die Strömungsrichtung der Milch umgebogen ist und eine Oeffnung aufweist. Das Ende 3 ist vorzugsweise in der Region des Zentrums der Milchförderleitung 1 angeordnet. Das andere Ende 4 des Absaugstutzens 2 mündet in eine elastische Saugleitung 5, an deren dem Absaugstutzen abgewandten Ende eine Injektionsnadel 6 vorhanden ist. Ein Schenkel 30 eines L-förmigen Supports 29 ist aussen an der Milchförderleitung im wesentlichen parallel zum Absaugstutzen 2 verlaufend, befestigt. Der andere Schenkel 31 des L-förmigen Supports steht im wesentlichen rechtwinklig zum Schenkel 30. Der Support 29 weist eine teilkreisförmige Innenfläche 32 zum Umleiten der flexiblen Saugleitung 5 auf. Eine an und für sich bekannte Schlauchpumpe 7 wirkt in die teilkreisförmige Innenfläche eingreifend auf die flexible Saugleitung. Die Schlauchpumpe 7 ist über eine Welle 28 durch einen Motor 27 angetrieben. Am Ende des Schenkels 31 ist ein Tragfuss 16 vorhanden, auf dessen dem Schenkel 31 abgewandten Seite ein topfförmiger Federkäfig 17 befestigt ist. Eine Spiralfeder 18 ist vom unten offenen Ende des Federkäfigs 17 in letzteren eingeführt und in nicht dargestellter Weise im Käfig gehalten. Durch eine in dieser Figur ebenfalls nicht dargestellte Fördereinrichtung auf ein anhebbar und absenkbares Teller geführte Probeentnahmeflasche 8 mit einem am oberen Ende angeordneten elastischen und selbstverschliessbaren Verschluss 21 drückt, währenddem die genannte Injektionsnadel im angehobenen Zustand des Tellers 15 den Verschluss durchdringt, gegen die Spiralfeder 18. Das Teller 15, das hydraulisch oder vorzugsweise pneumatisch über einen nur teilweisen angedeuteten Zylinder 22 anhebbar und absenkbar ist, umfasst ein Feststellorgan 19 zum Feststellen einer richtig positionierten Probeentnahmeflasche 8 auf dem Teller und ein Halteelement 20, vorzugsweise einen Dauer- oder Elektromagneten zum Halten der Flasche. Es ist dabei selbstverständlich, dass zumindest der Boden der Flasche aus einem magnetischen Werkstoff besteht. Das Teller 15, das Feststellorgan 19, das beispielsweise aus einem kapazitiven Näherungsindikator bestehen kann, das Halteelement 20 und der Pneumatikzylinder 22 sind zusammenfassend mit dem Bezugszeichen 10 als Zuführmittel bezeichnet. Die Spiralfeder 18 unterstützt beim Absenken des Tellers 15 den Abstreifvorgang der Injektionsnadel 6 aus dem Flaschenverschluss 21. Der Tragfuss 16 mit dem Federkäfig 17 und der Feder 18 sind als Niederhalteeinrichtung eines Abstreifmittels 11 zusammenfassend gekennzeichnet. Am unteren Ende der nicht bewegbaren Injektionsnadel 6 kann in einer besonderen Ausführungsform ein Sensor 14, vorzugsweise eine Lichtschranke mit einem Lichtsender und einem Lichtempfänger angeordnet sein. Der Lichtsender 23 ist über eine erste mehradrige elektrische Leitung 25 mit einem Steuermittel 9 verbunden. Eine gleichartige Verbindung (26) besteht zwischen dem Lichtempfänger (24) und dem Steuermittel 9. Letzteres, das ein Rechenmittel 12 mit einem zugeordneten Speichermittel 13 umfasst, dient u.a. auch zum Steuern der Schlauchpumpe 7. Dazu ist der Motor 27 über eine dritte elektrische Leitung 33 mit dem Steuermittel 9 verbunden.

Diese Vorrichtung, anhand der nachstehend das erfindungsgemässe Verfahren im Detail beschrieben wird, zeichnet sich durch ihren einfachen, einen sicheren Betrieb gewährleistenden, konstruktiven Aufbau aus.

Gegenüber bekannten Ausführungsarten fällt auf, dass die Injektionsnadel 6 unbewegbar angeordnet ist. Zum Entfernen von Milchresten 40 des Vorlieferanten, welche insbesondere innerhalb der Saugleitung 5 und der Injektionsnadel 6 anhaften können, wird nach dem erfindungsgemässen Verfahren wie folgt vorgegangen: sobald bei einer Milchübernahme die Förderleitung 1 mit der Milch des gegenwärtigen Lieferanten gefüllt ist, schaltet das Rechenmittel 12 über die dritte elektrische Leitung 33, den Motor 27 und die Schlauchpumpe 7 ein. Diese beginnt, Milch aus der Förderleitung über den Absaugstutzen 2 in die Saugleitung 5 und in die Injektionsnadel 6 zu pumpen. Zu diesem Zeitpunkt ist der Teller 15 mit der bereits darauf angeordneten Probeentnahmeflasche 8 noch in seiner abgesenkten Stellung, welche in der Fig. 1 gestrichelt angedeutet ist. Die Injektionsnadel 6 hat noch keinen Kontakt mit dem Flaschenverschluss 21. Die Schlauchpumpe 7 fördert solange Milch in die Saugleitung und die Injektionsnadel, bis deren Volumen vollständig mit Milch gefüllt sind. In einem ersten, bevorzugten Ausführungsbeispiel ist zum Ueberwachen des Füllzustandes kein Sensor 14 vorgesehen. Die bekannten Volumen der Schlauchleitung 5 und der Injektionsnadel 6 sind in den Speichermitteln 13, die dem Rechenmittel 12 zugeordnet sind, abgespeichert. Durch ein Software-Programm wird die Pumpe mit einer entsprechenden Drehzahl, die einer bestimmten Förderleistung entspricht, nun solange angesteuert, bis sie genau die Menge der Milch gefördert hat, die zum Füllen der genannten Volumen notwendig ist. Sobald die genannten Volumen vollständig gefüllt sind, wird die Schlauchpumpe 7 kurzzeitig angehalten und unmittelbar darauf in entgegengesetzter Richtung wieder in Betrieb gesetzt. Die vorgängig in die Saugleitung 5 und in die Injektionsnadel 6 gepumpte Milch wird nun zurück in die Förderleitung befördert. Auf diese Weise ist es äusserst einfach möglich, von einem Vorlieferanten anhaftende Milch aus den genannten Volumen zu entfernen. Es geht keine Milch verloren und es ist nicht notwendig, durch zusätzliche, konstruktive Massnahmen, Trichter zur Aufnahme von Spülmilch zuzuführen oder die Injektionsnadel über einen Behälter zu schwenken, um Spülmilch abzugeben. Erst nach diesem geschilderten Spülvorgang, der nur einige wenige Zehntelssekunden dauert, wird die Probeentnahmeflasche 8 durch den Teller 15 angehoben. Die Injektionsnadel 6 sticht durch den Flaschenverschluss 21. Die Spiralfeder 18 drückt dabei gegen das obere Ende des Flaschenverschlusses, um das Festhalten der Flasche 8 durch das Festhalteelement 20 zu unterstützen. Jetzt kann mit der eigentlichen Probeentnahme begonnen werden. Die Schlauchpumpe 7 wird dazu in der entsprechenden Drehrichtung eingeschaltet. Idealerweise sollte die Probeentnahme proportional über den ganzen Milchfördervorgang verlaufen. Anhand der Menge, der zu übernehmenden Milch, welche Grösse dem Rechenmittel 12 eingegeben worden ist, errechnet das Letztere die dazu erforderliche Fördermenge der Schlauchpumpe 7. Die durch den geschilderten Spülvorgang verlorengegangene Zeit von höchstens 0,5 Sekunden wird bei der Probeentnahme kompensiert, indem im ersten Moment die Schlauchpumpe 7 ein wenig mehr fördert, als erforderlich wäre. Am Ende der Probeentnahme (die Volumen der Schlauchleitung 5 und der Injektionsnadel 1 sind geleert) wird der Teller 15 mit der Probeentnahmeflasche 8 abgesenkt. Bei diesem Vorgang wird die Injektionsnadel 6 vom elastischen, sich selbst verschliessenden Flaschenverschluss abgestreift. Dieser Abstreifvorgang wird durch die Spiralfeder 18 der Niederhalteeinrichtung 16, 17, 18 unterstützt.

Selbstverständlich wäre es auch möglich, insbesondere den Füllvorgang beim Füllen und Entleeren der Volumen der Saugleitung 5 und der Injektionsnadel 6 mittels einem Sensor, der mit dem Bezugszeichen 14 angedeutet ist, zu überwachen. Dies könnte vorzugsweise mit einer Lichtschranke so erfolgen, dass der vom Lichtsender 23 ausgesendete Lichtstrahl unterbrochen wird, sobald ein Tropfen Milch in der Oeffnung der Injektionsnadel 6 erscheint. Der Lichtempfänger 24 könnte diesen Lichtstrahlunterbruch dem Rechenmittel 12 zurückmelden, welches sofort das Stoppen der Schlauchpumpe veranlassen würde.

Bei dieser Vorrichtung mit einer Schlauchpumpe 7 ist es nicht möglich, dass bei Vakuumschlägen in der Milchförderleitung 1 Milch aus der Probeentnahmeflasche 8 in die Förderleitung 1 zurückgelangt. Nebenbei sei noch erwähnt, dass beim Füllen der Probeentnahmeflasche 8 die sich darin befindende Luft über eine aussen an der Injektionsnadel 6 angeordnete Nut 40 entweichen kann.

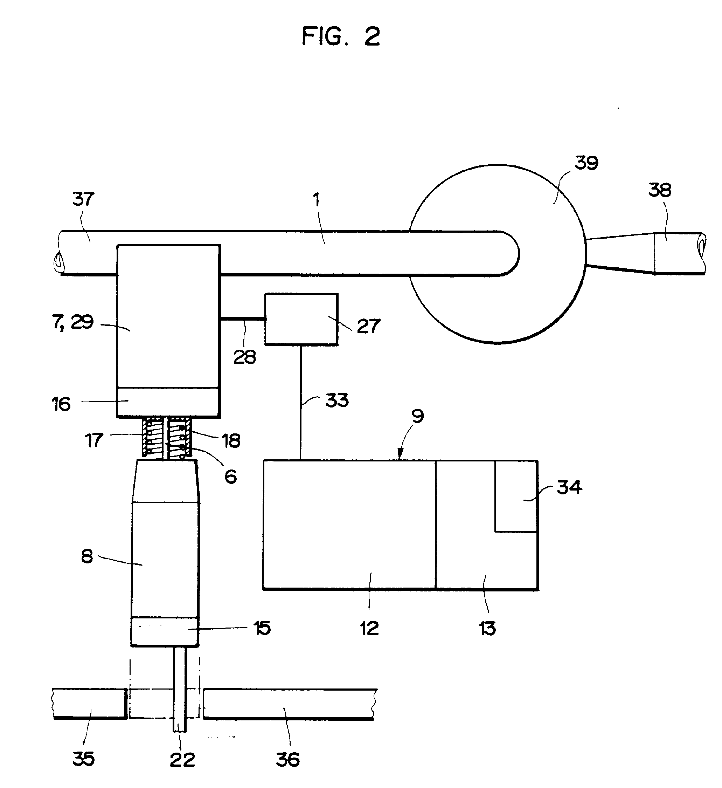

Eine Probenahmeeinrichtung, bei der die vorgängig beschriebene, erfindungsgemässe Vorrichtung zum Entnehmen von Milchproben an der Förderleitung 1 angeordnet ist, ist in der Fig. 2 dargestellt. Das eine Ende 37 der Förderleitung 1 ist mit einem nicht gezeigten Schlauch verbunden, der zum Einführen in den Lagertank des Milchlieferanten bestimmt ist. Das andere Ende 38 der Milchförderleitung 1 ist mit dem ebenfalls nicht gezeigten Tank des Milchsammelwagens verbunden, auf welchem die Probenahmeeinrichtung vorzugsweise angeordnet ist. Eine Förderpumpe 39, die in nicht weiter dargestellter Art, ebenfalls vom Steuermittel 9 angesteuert wird, fördert die Milch durch die Milchförderleitung 1. Mit 35 und 36 sind Fördermittel angedeutet, die in bekannter Art, beispielsweise mittels Förderbändern, arbeiten und zum Zu- und Abführen von Probeentnahmeflaschen 8 auf und vom Teller 15 in seiner abgesenkten Stellung bestimmt sind.

Um repräsentative Proben zu erhalten, ist es bisher üblich gewesen, die zu erwartende Milchmenge bei jedem Lieferanten über eine Tastatur oder andere Schaltmittel manuell in die Steuermittel 9 einzugeben. Da an der beschriebenen Vorrichtung bereits Rechenmittel 12 und Speichermittel 13 vorhanden sind, ist es vorteilhaft, die zu erwartende Milchmenge eines jeden Lieferanten an dafür vorgesehenen Speicherplätzen 34 abzuspeichern. Das Bedienungspersonal muss dann nur noch die Lieferantenkennung in die Rechenmittel eingeben, was heute vorzugsweise mit einer codierten Karte über einen nicht dargestellten und mit dem Rechenmittel 12 verbundenen Kartenleser erfolgen kann. Falsche Milchmengenvorgaben sind bei diesem System ausgeschlossen und nicht repräsentative Proben können kaum entnommen werden.

## Patentansprüche

1. Verfahren zum Entnehmen einer Milchprobe aus einer Milchförderleitung (1) in welche ein Ende (3) eines Absaugstutzen (2) eingeführt ist an dessen anderem Ende (4) eine Saugleitung (5) mit einer Injektionsnadel (6) angeordnet ist, wobei eine Schlauchpumpe (7) auf die Saugleitung wirkt, wobei die Volumen der Saugleitung und der Injektionsnadel vollständig mit Milch gefüllt und nach dem Füllvorgang wiederum entleert werden, indem die Milch in umgekehrter Richtung durch die Saugleitung in die Förderleitung zurückfliesst, und dass erst nach diesem Spülvorgang die Nadel in eine zugeführte Probeentnahmeflasche (8) eingeführt und mit der Probeentnahme begonnen wird.

2. Verfahren nach Anspruch 1, dadurch gekennzeichnet, dass das Füllen und Entleeren der genannten Volumen durch entsprechende Steuerung der Drehrichtung der Schlauchpumpe ausgeführt wird.

3. Verfahren nach Anspruch 1 oder 2, dadurch gekennzeichnet, dass die Probeentnahmeflasche zum Einführen der Injektionnadel angehoben wird.

4. Verfahren nach einem der Ansprüche 1 bis 3, dadurch gekennzeichnet, dass zum Füllen und Entleeren der genannten Volumen höchstens 0,5 Sekunden benötigt werden, und dass diese für die Probeentnahme verlorengegangene Zeit durch eine kurzzeitige Erhöhung der Förderleistung der Schlauchpumpe kompensiert wird.

5. Vorrichtung an einer Milchförderleitung (1) in welche ein Ende (3) eines Absaugstutzen (2) eingeführt ist an dessen anderem Ende (4) eine Saugleitung (5) mit einer Injektionsnadel (6) angeordnet ist, wobei eine Schlauchpumpe (7) auf die Saugleitung wirkt, und Mitteln zum Steuern (9) der Schlauchpumpe (7), vorhanden und so ausgestattet sind, daß wobei die Volumen der Saugleitung und der Injektionsnadel vollständig mit Milch gefüllt und nach dem Füllvorgang wiederum entleert werden, indem die Milch in umgekehrter Richtung durch die Saugleitung in die Förderleitung zurückfliesst, und dass erst nach diesem Spülvorgang die Nadel in eine zugeführte Probeentnahmeflasche (8) eingeführt und mit der Probeentnahme begonnen wird wobei weiter Mittel zum Zuführen (10) und Abstreifen (11) der Probeentnahmeflasche (8) vorhanden sind.

6. Vorrichtung nach Anspruch 5, dadurch gekennzeichnet, dass die Steuermittel (9) ein Rechenmittel (12) umfassen, welches zum Steuern und Ueberwachen des Füll- und Entleervorganges der genannten Volumen sowie der eigentlichen Probeentnahme bestimmt ist.

7. Vorrichtung nach Anspruch 6, dadurch gekennzeichnet, dass dem Rechenmittel (12) ein Speichermittel (13) zugeordnet ist, und dass die Grössen der genannten Volumen im Speichermittel zum Festlegen der Fördermenge der Schlauchpumpe beim Füll- und/oder Entleervorgang, gespeichert sind.

8. Vorrichtung nach Anspruch 5 oder 6 dadurch gekennzeichnet, dass an die Steuermittel (9) ein Sensor (14) zum Feststellen der vollständigen Füllung der genannten Volumen angeschlossen ist.

9. Vorrichtung nach einem der Ansprüche 5 bis 8, dadurch gekennzeichnet, dass die Zuführmittel ein anhebbares Teller (15) und die Abstreifmittel (11) eine Niederhalteeinrichtung (16, 17, 18) umfassen.

10. Vorrichtung nach Anspruch 9, dadurch gekennzeichnet, dass das Teller (15) pneumatisch oder hydraulisch anhebbar ist, ein Organ zum Feststellen (20) des Vorhandenseins einer Probeentnahmeflasche sowie Elemente zum Halten der Flasche aufweist.

11. Vorrichtung nach Anspruch 9 oder 10, dadurch gekennzeichnet, dass an der Niederhalteeinrichtung eine im angehobenen Zustand der Probeentnahmeflasche (8) gegen den Flaschenverschluss (21) wirkende Spiralfeder (18) angeordnet ist.

12. Probenahmeeinrichtung, insbesondere an Milchsammelwagen, mit einer Vorrichtung nach Patentanspruch 5, dadurch gekennzeichnet, dass die Steuermittel einen Milchmengenspeicher (34) zum Speichern der voraussichtlichen Milchmengen der einzelnen Lieferanten aufweisen.

13. Einrichtung nach Anspruch 12, dadurch gekennzeichnet, dass durch die gespeicherte voraussichtliche Milchmenge eines Lieferanten die Förderleistung der Schlauchpumpe zum Erhalten einer repräsentativen Probeentnahme steuerbar ist.

## Claims

1. A method for taking a milk sample from a milk conveyor pipe (1) into which is inserted one end (3) of a suction connection piece (2) at whose other end (4) there is attached a suction pipe (5) with an injection syringe (6), a hose pump (7) interacting with the suction pipe, the volumes of the suction pipe and the injection syringe being completely filled with milk and emptied again after the filling procedure, in that the milk flows back in the opposite direction through the suction pipe into the conveyor pipe, and wherein the syringe is introduced into a supplied sample bottle (8) only after this flushing step and the sampling is begun.

2. A method according to claim 1, wherein the filling and emptying of the said volumes is carried out by appropriate control of the direction of rotation of the hose pump.

3. A method according to claim 1 or 2, wherein the sample bottle is raised to permit insertion of the injection syringe.

4. A method according to one of the claims 1 to 3, wherein 0.5 seconds at most is required to fill and empty the said volumes and wherein this time lost for sampling is compensated by an increase of short duration in the conveying capacity of the hose pump.

5. A device on a milk conveyor pipe (1) into which is inserted one end (3) of a suction connection piece (2) at whose other end (4) there is attached a suction pipe (5) with an injection syringe (6), a hose pump (7) interacting with the suction pipe, and means (9) for controlling the hose pump (7) being provided and equipped in such a way that the volumes of the suction pipe and the injection syringe are completely filled with milk and emptied again after the filling procedure, in that the milk flows back in the opposite direction through the suction pipe into the conveyor pipe, and wherein the syringe is introduced into a supplied sample bottle (8) only after this flushing step and the sampling is begun, further means being present for conveying (10) and withdrawal (11) of the sample bottle (8).

6. A device according to claim 5, wherein the control means (9) comprise a calculating device (12) which is intended to control and monitor the filling and emptying process of the said volumes as well as the actual sampling.

7. A device according to claim 6, wherein the calculating device (12) includes a memory (13) and wherein the amounts of the said volumes are stored in the memory to determine the quantity to be conveyed by the hose pump during the filling and/or emptying process.

8. A device according to claim 5 or 6, wherein a sensor (14) for detecting when the said volumes are completely full is connected to the control means (9).

9. A device according to one of the claims 5 to 8, wherein the conveyance means comprise a dish which can be raised (15) and the withdrawal means (11) comprise a holding down device (16, 17, 18).

10. A device according to claim 9, wherein the dish (15) can be raised pneumatically or hydraulically and has an element for determining (20) the presence of a sample bottle and elements for retaining the bottle.

11. A device according to claim 9 or 10, wherein a spiral spring (18) is positioned on the holding down device and acts against the bottle seal (21) when the sample bottle (8) is in the raised position.

12. Sampling equipment, particularly on milk collection lorries, with a device according to claim 5, wherein the control means have a milk quantity recorder (34) for recording the milk quantities foreseen from individual suppliers.

13. Equipment according to claim 12, wherein the conveying capacity of the hose pump to obtain a representative sample is controllable by means of the recorded, foreseen milk quantity of a supplier.

## Revendications

1. Procédé de prélèvement d'un échantillon de lait d'une conduite de transport de lait (1), dans laquelle est introduite une extrémité (3) d'un raccord d'aspiration (2), à l'autre extrémité (4) duquel est disposée une conduite d'aspiration (5) avec une seringue d'injection (6), une pompe péristaltique (7) agissant sur la conduite d'aspiration, le volume de la conduite d'aspiration et de la seringue d'injection étant entièrement rempli de lait et à nouveau vidé après le remplissage par le fait que le lait revient dans la conduite de transport dans la direction inverse par la conduite d'aspiration et que ce n'est qu'après ce processus de lavage que la seringue est introduite dans une bouteille de prélèvement d'échantillons (8) avancée et que le prélèvement d'échantillons commence.

2. Procédé selon la revendication 1, caractérisé en ce que le remplissage et la vidange du volume mentionné sont réalisés par la commande correspondante du sens de rotation de la pompe péristaltique.

3. Procédé selon la revendication 1 ou 2, caractérisé en ce que la bouteille de prélèvement d'échantillons est relevée pour introduire la seringue d'injection.

4. Procédé selon l'une des revendications 1 à 3, caractérisé en ce qu'au maximum 0,5 seconde est nécessaire pour le remplissage et la vidange du volume mentionné et que ce temps perdu pour le prélèvement d'échantillons est compensé par une augmentation momentanée du débit de la pompe péristaltique.

5. Dispositif sur une conduite de transport de lait (1) dans laquelle est introduite une extrémité (3) d'un raccord d'aspiration (2), à l'autre extrémité (4) duquel est disposée une conduite d'aspiration (5) avec une seringue d'injection (6), une pompe péristaltique (7) agissant sur la conduite d'aspiration et les moyens de commande (9) de la pompe péristaltique (7) qui existent et sont équipés de manière que le volume de la conduite d'aspiration et de la seringue d'injection entièrement rempli de lait est à nouveau vidé après le remplissage par le fait que le lait revient dans la conduite de transport dans la direction inverse par la conduite d'aspiration et que ce n'est qu'après ce processus de lavage que la seringue est introduite dans une bouteille de prélèvement d'échantillons (8) avancée et que le prélèvement d'échantillons commence, d'autres moyens existant pour avancer (10) et essuyer (11) la bouteille de prélèvement d'échantillons (8).

6. Dispositif selon la revendication 5, caractérisé en ce que les moyens de commande (9) comprennent un moyen de calcul (12), destiné à la commande et à la surveillance du processus de remplissage et de vidage du volume mentionné, de même que du prélèvement d'échantillons proprement dit.

7. Dispositif selon la revendication 6, caractérisé en ce qu'une mémoire (13) est attribuée au moyen de calcul (12) et que les grandeurs des volumes mentionnés sont mémorisées dans la mémoire pour la détermination du débit de la pompe péristaltique lors du remplissage et/ou du vidage.

8. Dispositif selon la revendication 5 ou 6, caractérisé en ce qu'un capteur (14) de détermination de l'intégralité du remplissage des volumes mentionnés est raccordé au moyen de commande (9).

9. Dispositif selon l'une des revendications 5 à 8, caractérisé en ce que les moyens d'avance comprennent un plateau relevable (15) et les moyens d'essuyage (11) un dispositif de retenue (16, 17, 18).

10. Dispositif selon la revendication 9, caractérisé en ce que le plateau (15) est relevable pneumatiquement ou hydrauliquement, présente un organe de détermination (20) de la présence d'une bouteille de prélèvement d'échantillons, de même que des éléments pour le maintien de la bouteille.

11. Dispositif selon la revendication 9 ou 10, caractérisé en ce qu'est disposé sur le dispositif de maintien un ressort hélicoïdal (18) agissant contre la fermeture de la bouteille (21) lorsque la bouteille de prélèvement d'échantillons (8) est relevée.

12. Dispositif de prélèvement d'échantillons, en particulier sur des installations de collecte de lait, doté d'un dispositif selon la revendication 5 du brevet, caractérisé en ce que les moyens de commande présentent une mémoire des quantités de lait (34) pour la mémorisation des quantités de lait prévisibles des différents fournisseurs.

13. Dispositif selon la revendication 12, caractérisé en ce que, du fait de la quantité de lait d'un fournisseur mémorisée prévisionnellement, le débit de la pompe péristaltique est commandable pour l'obtention d'un prélèvement d'échantillons représentatifs.
